Europäisches Patentamt

(19) European Patent Office

Office européen des brevets

(11) Numéro de publication : **0 065 054**
**B1**

(12) FASCICULE DE BREVET EUROPÉEN

(45) Date de publication du fascicule du brevet :
**22.10.86**

(21) Numéro de dépôt : **81401298.5**

(22) Date de dépôt : **12.08.81**

(51) Int. Cl.⁴ : **A 61 B 10/00**

(54) **Pince à biopsie.**

(30) Priorité : **06.05.81 FR 8109035**

(43) Date de publication de la demande :
**24.11.82 Bulletin 82/47**

(45) Mention de la délivrance du brevet :
**22.10.86 Bulletin 86/43**

(84) Etats contractants désignés :
**CH DE GB LI**

(56) Documents cités :
**CH-A-  547 103**
**FR-A- 1 378 088**
**FR-A- 2 253 490**
**US-A- 3 175 554**
**US-A- 3 404 677**

(73) Titulaire : **METALLISATIONS ET TRAITEMENTS OPTI-
QUES M.T.O.**
**11 rue Ampère**
**F-91300 Massy (FR)**

(72) Inventeur : **Regnier, Michel**
**28, Rue du Château D'Eau**
**F-91130 Ris Orangis (FR)**
Inventeur : **Hascoet, Martial**
**74, rue de Sèvres**
**F-75007 Paris (FR)**

(74) Mandataire : **Kedinger, Jean-Paul et al**
**c/o Cabinet Maiemont 42, avenue du Président Wilson**
**F-75116 Paris (FR)**

## Description

La présente invention concerne une pince à biopsie dont le corps comprend un tube extérieur, un ensemble intérieur enfilé dans le tube extérieur, des mâchoires opposées prévues à l'extrémité antérieure de l'ensemble intérieur, ces mâchoires s'avançant en avant de l'extrémité antérieure du tube extérieur en s'écartant l'une de l'autre et étant déplaçables entre une position ouverte dans laquelle elles sont éloignées de l'axe longitudinal du tube extérieur et une position fermée dans laquelle elles se rejoignent sur cet axe, et des moyens de commande pour déplacer l'un par rapport à l'autre axialement le tube extérieur et l'ensemble intérieur et permettre ainsi au tube extérieur de déplacer les mâchoires entre leurs positions ouverte et fermée.

FR-A-1 378 088 décrit une pince à biopsie de ce type dans laquelle l'ensemble intérieur est constitué par deux tiges métalliques dont l'extrémité antérieure de chacune d'elles constitue une mâchoire. Or comme les deux tiges occupent tout l'espace délimité à l'intérieur du tube extérieur, cette pince ne peut être enfilée sur le corps d'un endoscope de contact à usage médical ou vétérinaire, afin de pouvoir réaliser des prélèvements de fragments de tissus tout en procédant à un contrôle visuel de l'intervention.

L'invention selon la présente demande a précisément pour objet de remédier à cet inconvénient et, pour ce faire, elle a pour objet une pince à biopsie, qui se caractérise en ce que l'ensemble intérieur est constitué par un second tube s'étendant coaxialement avec le tube extérieur, et en ce que les mâchoires sont constituées par deux languettes diamétralement opposées prolongeant l'extrémité antérieure du tube intérieur, ces languettes se terminant par des dents réalisées à l'extrémité libre d'une partie coudée sensiblement à angle droit en direction de l'axe longitudinal des deux tubes.

Grâce à sa structure tubulaire, cette pince à biopsie peut être enfilée sur le corps d'un endoscope de contact, de sorte que le choix du fragment du tissu à prélever, l'exécution du prélèvement et l'extraction de celui-ci peuvent avoir lieu sous un contrôle visuel permanent, ce qui facilite la tâche du praticien et permet une exécution précise et sûre de l'intervention.

Par ailleurs, comme ses mâchoires se terminent par une partie coudée sensiblement à angle droit, l'extrémité distale de l'endoscope peut maintenant demeurer à leur contact lors de leur fermeture. Le praticien a de ce fait la possibilité de voir le fragment de tissu pendant le prélèvement et ne risque donc plus de prélever un fragment autre que celui qu'il avait sélectionné lorsque les mâchoires étaient ouvertes.

D'autre part, ses mâchoires sont dépourvues de parties saillantes effilées, ce qui élimine les risques de blessures accidentelles lors de leur introduction dans la cavité.

Avantageusement, chaque languette comporte, entre sa partie coudée et sa liaison avec le tube intérieur, un renflement coopérant avec une encoche longitudinale réalisée à l'extrémité antérieure du tube extérieur, ce renflement faisant saillie à l'extérieur de l'encoche lorsque les mâchoires sont ouvertes et s'étendant dans l'encoche lorsque les mâchoires sont fermées.

Ainsi, les mâchoires ne font pas saillie sur la paroi latérale du tube extérieur lorsqu'elles sont fermées, ce qui réduit encore les risques de blessures.

Selon un mode de réalisation particulier de la pince conforme à l'invention, les moyens de commande sont constitués par deux bras de préhension articulés l'un par rapport à l'autre et reliés à l'extrémité postérieure des deux tubes, l'un de ces bras étant solidaire du tube extérieur tandis que l'autre est articulé sur le second tube, lequel se prolonge au-delà de l'extrémité postérieure du tube extérieur.

Un mode d'exécution de la présente invention sera décrit ci-après à titre d'exemple nullement limitatif en référence aux dessins annexés dans lesquels :

La figure 1 est une vue latérale d'une pince à biopsie conforme à l'invention.

La figure 2 est une vue en perspective et à échelle agrandie de l'extrémité antérieure du tube extérieur de la pince, les mâchoires de celle-ci étant représentées ouvertes.

La figure 3 est une vue analogue à la figure 2 mais dans laquelle les mâchoires sont représentées fermées.

La figure 4 est une vue partielle en coupe axiale et à échelle agrandie de la pince visible sur la figure 1.

La figure 5 est une vue en coupe effectuée suivant la ligne V-V de la figure 4 ; et

La figure 6 est une vue en coupe axiale de l'extrémité antérieure de la pince visible sur la figure 4 mais dans laquelle les mâchoires sont représentées fermées.

La pince à biopsie que l'on peut voir sur les dessins comporte un tube extérieur 1, un tube intérieur 2 enfilé dans le tube extérieur avec lequel il s'étend coaxialement, deux mâchoires 3 faisant saillie sur l'extrémité antérieure 4 du tube extérieur, et des moyens de commande 5 pour déplacer axialement l'un des deux tubes et permettre simultanément l'actionnement des mâchoires.

Dans l'exemple représenté, les moyens de commande 5 sont prévus pour déplacer axialement le tube intérieur dans le tube extérieur. Mais il va de soi qu'ils pourraient déplacer le tube extérieur sur le tube intérieur.

Le tube intérieur a un diamètre extérieur qui est légèrement plus petit que le diamètre intérieur du tube extérieur. Il se prolonge au-delà de l'extrémité postérieure 6 de celui-ci et se termine par une partie filetée 7 dont le rôle sera précisé ci-après.

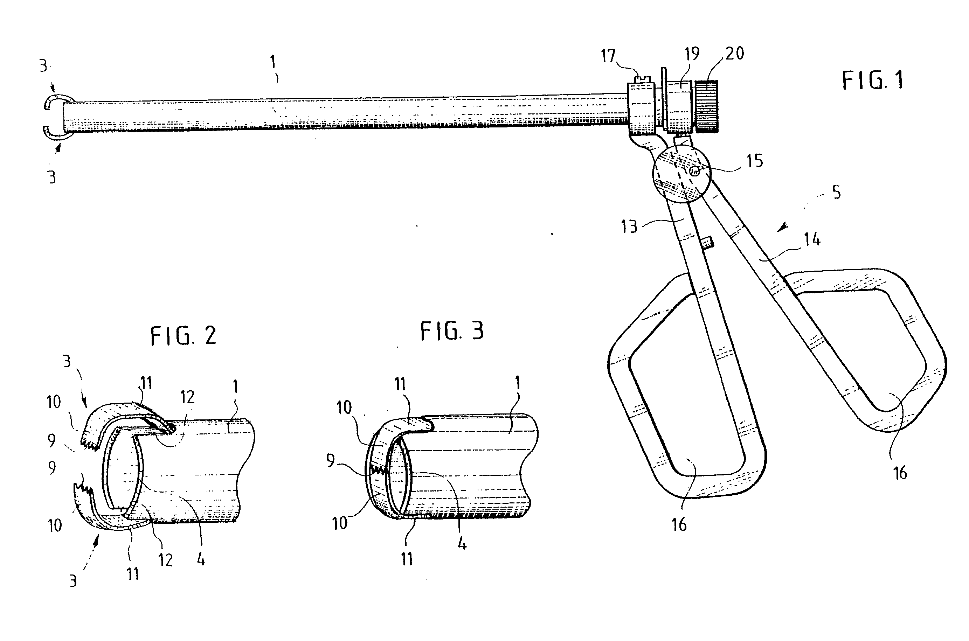

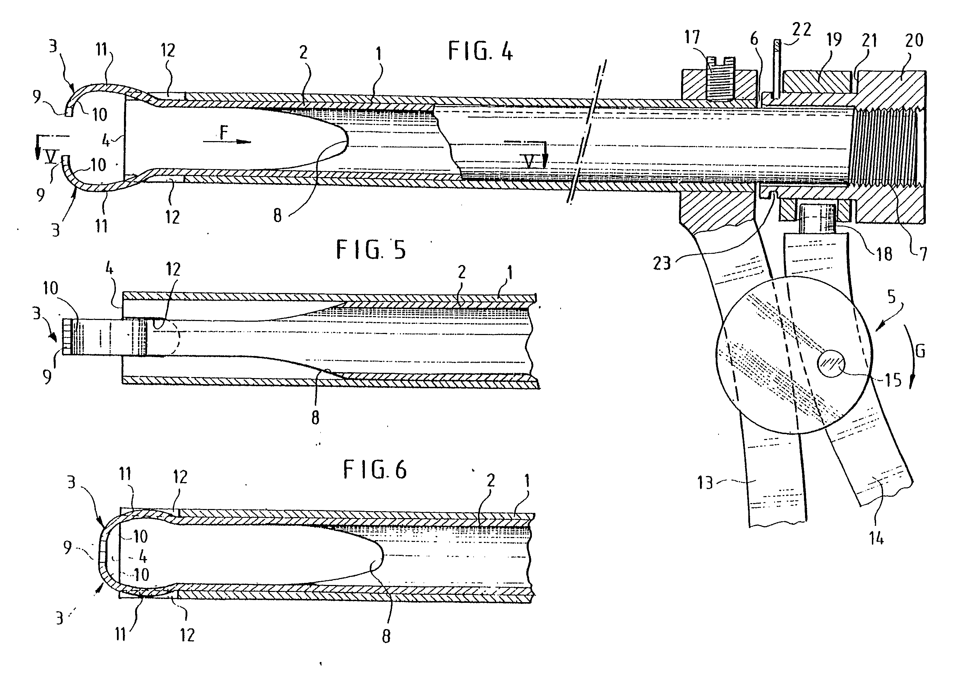

Les mâchoires sont déplaçables entre une position ouverte (visible sur les figures 1, 2 et 4) dans laquelle elles sont éloignées de l'axe longitudinal des deux tubes et une position fermée (visible sur les figures 3 et 6) dans laquelle elles se rejoignent sur cet axe. Elles sont portées par l'extrémité antérieure 8 du tube intérieur et se prolongent en direction de l'extrémité antérieure 4 du tube extérieur en s'écartant légèrement l'une de l'autre. Ainsi, grâce à leur forme divergente, le tube extérieur assurera leur fermeture lors du recul du tube intérieur et leur permettra de s'ouvrir d'elles-mêmes sous l'effet de leur élasticité lorsque le tube intérieur sera ramené dans sa position initiale.

Dans l'exemple de réalisation représenté sur les dessins, les mâchoires sont constituées par deux languettes diamétralement opposées prolongeant l'extrémité antérieure 8 du tube intérieur. Elles se terminent par des dents 9 réalisées à l'extrémité d'une partie 10 coudée sensiblement à angle droit en direction de l'axe longitudinal des tubes 1 et 2. Elles comportent en outre chacune, entre leur partie coudée 10 et leur liaison avec le tube intérieur, un renflement 11 coopérant avec une encoche longitudinale 12 réalisée à l'extrémité antérieure 4 du tube extérieur. Comme le montrent les figures 4 et 6, les renflements 11 sont conformés de manière à faire saillie à l'extérieur des encoches 12 lorsque les mâchoires sont ouvertes et à s'étendre dans les encoches lorsque celles-ci sont fermées.

Quant aux moyens de commande 5, ils sont constitués par deux bras de préhension 13 et 14 articulés l'un sur l'autre autour d'un axe 15 et dont les extrémités inférieures se terminent par deux boucles 16 destinées à recevoir respectivement le pouce et l'index de l'utilisateur de la pince.

En se référant plus particulièrement à la figure 4, on remarquera que l'extrémité supérieure du bras 13 est enfilée sur le tube extérieur 1 et est immobilisée sur celui-ci au moyen d'un goujon fileté 17. On remarquera également que l'extrémité supérieure du bras 14 se termine par un téton 18 inséré dans un perçage réalisé dans une douille 19. Cette dernière, qui est enfilée sur un manchon 20 vissé sur la partie filetée 7 du tube intérieur, est immobilisée entre un épaulement 21 du manchon et une bague élastique amovible 22 retenue dans une gorge 23 réalisée dans ce dernier.

La configuration particulière des extrémités supérieures des deux bras de préhension et des pièces assurant leur retenue sur les deux tubes permet un montage et un démontage aisés et rapides de la pince, en vue du nettoyage de celle-ci.

Pour démonter la pince, il suffit en effet d'enlever la bague 22, de dévisser le manchon 20 pour le séparer de la partie filetée 7 du tube intérieur et d'extraire celui-ci du tube extérieur en le tirant depuis l'extrémité antérieure de ce dernier. Pour remonter la pince, il suffit bien entendu d'effectuer les mêmes opérations, mais en sens inverse.

Le fonctionnement de la pince selon la présente invention est très simple. Pour fermer les mâchoires, l'opérateur fait pivoter le bras 14 dans le sens de la flèche G autour de l'axe 15. Pendant que le bras 14 pivote, son téton 18 pousse la douille 19 et le manchon 20 vers l'arrière, dans le sens de la flèche F. Comme le tube intérieur 2 est solidaire du manchon, il se déplace à son tour dans le sens de la flèche F. Pendant ce temps, les renflements 11 des mâchoires 3 glissent contre la surface interne du tube extérieur 1, laquelle les contraint à se rétracter dans les encoches 12 et permet par suite aux parties coudées 10 de se rencontrer sur l'axe longitudinal des deux tubes.

Pour ouvrir les mâchoires 3, il suffit de faire pivoter le bras 14 en sens inverse. Au fur et à mesure que celui-ci pivote autour de l'axe 15, le tube intérieur se déplace vers l'avant tandis que les mâchoires s'éloignent l'une de l'autre sous l'effet de leur élasticité, leurs renflements 11 faisant saillie hors des encoches 12 et leur permettant ainsi de s'écarter l'une de l'autre.

**Revendications**

1. Pince à biopsie dont le corps comprend un tube extérieur (1), un ensemble intérieur (2) enfilé dans le tube extérieur (1), des mâchoires opposées (3) prévues à l'extrémité antérieure (8) de l'ensemble intérieur (2), ces mâchoires (3) s'avançant en avant de l'extrémité antérieure (4) du tube extérieur (1) en s'écartant l'une de l'autre et étant déplaçables entre une position ouverte dans laquelle elles sont éloignées de l'axe longitudinal du tube extérieur (1) et une position fermée dans laquelle elles se rejoignent sur cet axe, et des moyens de commande (5) pour déplacer l'un par rapport à l'autre axialement le tube extérieur (1) et l'ensemble intérieur (2) et permettre ainsi au tube extérieur (1) de déplacer les mâchoires (3) entre leurs positions ouverte et fermée, caractérisée en ce que l'ensemble intérieur est constitué par un second tube (2) s'étendant coaxialement avec le tube extérieur (1), et en ce que les mâchoires (3) sont constituées par deux languettes diamétralement opposées prolongeant l'extrémité antérieure (8) du tube intérieur, ces languettes se terminant par des dents (9) réalisées à l'extrémité libre d'une partie (10) coudée sensiblement à angle droit en direction de l'axe longitudinal des deux tubes (1, 2).

2. Pince à biopsie selon la revendication 1, caractérisée en ce que chaque languette comporte, entre sa partie coudée (10) et sa liaison avec le tube intérieur (2), un renflement (11) coopérant avec une encoche longitudinale (12) réalisée à l'extrémité antérieure (4) du tube extérieur (1), ce renflement faisant saillie à l'extérieur de l'encoche lorsque les mâchoires (3) sont ouvertes et s'étendant dans l'encoche lorsque les mâchoires sont fermées.

3. Pince à biopsie selon la revendication 1 ou 2, caractérisée en ce que les moyens de commande (5) sont constitués par deux bras de préhension (13, 14) articulés l'un par rapport à l'autre et reliés à l'extrémité postérieure des deux tubes (1, 2), l'un (13) de ces bras étant solidaire du tube extérieur (1) tandis que l'autre (14) est articulé sur le second tube (2), lequel se prolonge au-delà de l'extrémité postérieure du tube extérieur (1).

**Claims**

1. Biopsy forceps, the body of which comprises an outer tube (1), an inner assembly (2) fitted into the outer tube (1), opposing jaws (3) provided at the front end (8) of the inner assembly (2), these jaws (3) projecting forward from the front end (4) of the outer tube (1) while being spaced apart from each other and being movable between an open position in which they are distant from the longitudinal axis of the outer tube (1) and a closed position in which they come into contact along this axis, and control means (5) for axially moving the outer tube (1) and the inner assembly (2) with respect to each other and thus permitting the outer tube (1) to move the jaws (3) between their open and closed positions, characterized in that the inner assembly is formed by a second tube (2) extending coaxially with the outer tube (1), and in that the jaws (3) are formed by two diametrically opposed tongues extending the front end (8) of the inner tube, these tongues ending in teeth (9) formed at the free end of a part (10) bent substantially at right angle in the direction of the longitudinal axis of both tubes (1, 2).

2. Biopsy forceps according to claim 1, characterized in that each tongue comprises, between its bent part (10) and its connection with the inner tube (2), a swelling (11) cooperating with a longitudinal notch (12) formed at the front end (4) of the outer tube (1), this swelling projecting outside the notch when the jaws (3) are open and extending into the notch when the jaws are closed.

3. Biopsy forceps according to claim 1 or 2, characterized in that the control means (5) are formed by two gripping arms (13, 14) pivotally coupled to each other and connected to the rear end of both tubes (1, 2), one (13) of these arms being integral with the outer tube (1) while the other (14) is pivotally fitted on the second tube (2), which extends beyond the rear end of the outer tube (1).

**Patentansprüche**

1. Biopsiezange, deren Körper ein Außenrohr (1), eine in das Außenrohr (1) eingeführte innere Baugruppe (2), am vorderen Ende (8) der inneren Baugruppe (2) einander gegenüber angeordnete Backen (3), welche vorwärts des vorderen Endes (4) des Außenrohrs (1) auseinanderstreben und zwischen einer geöffneten Stellung, in welcher sie von der Mittelachse des Außenrohrs (1) entfernt sind, und einer geschlossenen Stellung bewegbar sind, in welcher sie in der genannten Achse zusammentreffen, und eine Betätigungseinrichtung (5) zum axialen Bewegen des Außenrohrs (1) und der inneren Baugruppe (2) relativ zueinander aufweist, wobei die Backen (3) durch das Außenrohr (1) zwischen ihrer geöffneten und ihrer geschlossenen Stellung bewegbar sind, dadurch gekennzeichnet, daß die innere Baugruppe durch ein zweites Rohr (2) gebildet ist, welches sich koaxial mit dem Außenrohr (1) erstreckt, und daß die Backen (3) durch zwei einander diametral gegenüberstehende, das vordere Ende (8) des Innenrohrs verlängernde Zungen gebildet sind, welche in Zähnen (9) am freien Ende eines im wesentlichen im rechten Winkel auf die Längsachse der beiden Rohre (1, 2) abgebogenen Abschnitts (10) auslaufen.

2. Biopsiezange nach Anspruch 1, dadurch gekennzeichnet, daß jede Zunge zwischen ihrem abgewinkelten Abschnitt (10) und ihrer Verbindung mit dem Innenrohr (2) eine Ausbauchung (11) hat, welche mit einem am vorderen Ende (4) des Außenrohrs (1) ausgebildeten, in Längsrichtung verlaufenden Ausschnitt (12) zusammenwirkt, wobei die Ausbauchung bei geöffneten Bakken (3) aus dem Ausschnitt hervorsteht und sich bei geschlossenen Backen in dem Ausschnitt erstreckt.

3. Biopsiezange nach Anspruch 1 oder 2, dadurch gekennzeichnet, daß die Betätigungseinrichtung (5) aus zwei Griffhebeln (13, 14) gebildet ist, welche gelenkig miteinander verbunden sowie mit dem hinteren Ende der beiden Rohre (1, 2) verbunden sind, wobei der eine Hebel (13) fest mit dem Außenrohr (1) verbunden ist, während der andere (14) gelenkig mit dem zweiten Rohr (2) verbunden ist, welches sich über das hintere Ende des Außenrohrs (1) hinaus verlängert.

FIG.1

FIG. 2

FIG. 3

FIG. 4

FIG. 5

FIG. 6

0 065 054